(19) **Europäisches Patentamt / European Patent Office / Office européen des brevets**

(11) **EP 1 973 420 B1**

(12) **EUROPEAN PATENT SPECIFICATION**

(45) Date of publication and mention
of the grant of the patent:
**22.04.2015 Bulletin 2015/17**

(51) Int Cl.:
*A23L 1/05* $^{(2006.01)}$    *A23L 1/29* $^{(2006.01)}$
*A23L 1/305* $^{(2006.01)}$    *A23L 1/035* $^{(2006.01)}$
*A23L 1/0524* $^{(2006.01)}$    *A23L 1/0532* $^{(2006.01)}$
*A23L 1/054* $^{(2006.01)}$    *A61K 9/107* $^{(2006.01)}$

(21) Application number: **06847785.0**

(22) Date of filing: **19.12.2006**

(86) International application number:
**PCT/US2006/048430**

(87) International publication number:
**WO 2007/075683 (05.07.2007 Gazette 2007/27)**

(54) **INDUCED-VISCOSITY NUTRITIONAL EMULSIONS**

NÄHRSTOFFEMULSIONEN MIT INDUZIERTER VISKOSITÄT

EMULSIONS ALIMENTAIRES A VISCOSITE INDUITE

(84) Designated Contracting States:
**DE ES GB IE IT TR**

(30) Priority: **21.12.2005 US 752613 P**

(43) Date of publication of application:
**01.10.2008 Bulletin 2008/40**

(73) Proprietor: **ABBOTT LABORATORIES**
**Abbott Park, IL 60064-3500 (US)**

(72) Inventors:
• **LAI, Chron-si**
**Blacklick, OH 43004 (US)**
• **JOHNS, Paul W.**
**Columbus, OH 43221 (US)**

(74) Representative: **Modiano, Micaela Nadia et al**
**Modiano Josif Pisanty & Staub Ltd**
**Thierschstrasse 11**
**80538 München (DE)**

(56) References cited:
**US-A1- 2002 193 344    US-A1- 2003 013 679**

• **MURRAY S M ET AL: "APPARENT DIGESTIBILITY AND GLYCAEMIC RESPONSES TO AN EXPERIMENTAL INDUCED VISCOSITY DIETARY FIBRE INCORPORATED INTO AN ENTERAL FORMULA FED TO DOGS CANNULATED IN THE ILEUM" FOOD AND CHEMICAL TOXICOLOGY, XX, XX, vol. 37, January 1999 (1999-01), pages 47-56, XP000922559 ISSN: 0278-6915**

**Description**

**Technical Field**

The present invention relates to induced-viscosity nutritional emulsions having a protein component with a defined protein-bound methionine sulfoxide content for improved emulsion stability.

**Background of the Invention**

There are many different types of nutritional emulsions commercially available or are otherwise disclosed in the literature. These are typically oil-in-water emulsions comprising a balance of fat, protein, carbohydrate, vitamins, and minerals. Some examples include Glucerna® and Ensure® brands of packaged nutritional liquids, available from Abbott Laboratories, Columbus, Ohio.

Recently, a new type of nutritional liquid has been developed that contains, as part of a carbohydrate component, an induced viscosity fiber system. These liquids have a packaged viscosity typical of a nutritional emulsion, but because of the fiber system, result in a higher induced viscosity following consumption. The increased viscosity helps reduce gastric emptying and the subsequent blood glucose response. The viscosity increase within the stomach also provides a sense of fullness and enhanced satiety. These induced viscosity beverages are especially useful in diabetics and in people interested in maintaining or losing weight.

For example, U.S. Patent Application 20020193344 (Wolf et al.) discloses an induced viscosity beverage, wherein the beverage contains an induced viscosity fiber system having a soluble, anionic fiber in combination with a water insoluble, acid-soluble cation. The viscosity of the beverage increases following consumption when exposed to the low pH of the stomach.

In yet another example, U.S. Patent Application 20030013679 (Wolf et al.) discloses an induced viscosity beverage, wherein the beverage comprises a partially hydrolyzed starch in combination with a neutral soluble fiber. The viscosity of the beverage increases following consumption when exposed to acid and amylase in the stomach.

It has now been discovered that induced viscosity nutritional emulsions are more prone to creaming and protein sedimentation than conventional emulsions, even as soon as two weeks after formulation. It was also been discovered that this inherent instability can be significantly improved by formulating the induced viscosity emulsions with proteins having a protein-bound methionine sulfoxide content that is 8% or less of the total protein-bound methionine, on a molar basis.

**SUMMARY OF THE INVENTION**

The present invention is directed to induced viscosity nutritional emulsions comprising (A) protein having a protein-bound methionine sulfoxide (MSO) content that represents 8% or less, on a molar basis, of the total protein-bound methionine, (B) fat, and (C) an induced viscosity fiber system that provides the emulsion with a packaged viscosity of less than 300 centipoise (cps) and an induced viscosity following consumption of at least 300 cps. The nutritional emulsions are oil-in-water emulsions.

It has been discovered herein that the physical stability of an induced viscosity nutritional emulsion is inversely related to the methionine sulfoxide content of the protein ingredient in the emulsion. More specifically, it has been discovered that an improved physically stable liquid nutritional product is produced when a protein source with a methionine sulfoxide (MSO) content of 8% or less of the total protein-bound methionine, on a molar basis, is used in the induced viscosity nutritional emulsion.

**DETAILED DESCRIPTION OF THE INVENTION**

The induced viscosity nutritional emulsions of the present invention comprise as essential ingredients fat, selected proteins defined by a methionine sulfoxide content, and a carbohydrate component including an induced viscosity fiber system. These and other essential or optional elements or features of the nutritional emulsions of the present invention are described in detail hereinafter.

The term "nutritional emulsion" as used herein, unless otherwise specified, refers to oral liquids in the form of oil-in-water emulsions comprising fat, protein, and carbohydrates, which may be formulated as meal replacement products, nutritional supplements, or continuous (or intermittent) enteral feedings.

The term "induced viscosity nutritional emulsion" as used herein, unless otherwise specified, refers to a nutritional emulsion comprising an induced viscosity fiber system as defined herein.

The term "induced viscosity fiber system" as used herein, unless otherwise specified, means any fiber-containing material or composition, that when added to a nutritional emulsion allows for a drinkable emulsion viscosity (packaged viscosity) at room temperature and an increased (induced) emulsion viscosity following consumption.

The nutritional emulsions of the present invention may comprise, consist of, or consist essentially of the essential elements and limitations of the invention described herein, as well as any additional or optional ingredients, components, or limitations described herein or otherwise useful in a nutritional or pharmaceutical application.

All percentages, parts and ratios as used herein are by weight of the total composition, unless otherwise specified. All such weights as they pertain to listed ingredients are based upon the active level and, therefore, do not include solvents or by-products that may be included in commercially available materials, unless otherwise specified.

All numerical ranges as used herein, whether or not expressly preceded by the term "about", are intended and understood to be preceded by that term, unless otherwise specified.

All references to singular characteristics or limitations of the present invention shall include the corresponding plural characteristics or limitations, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The nutritional emulsions of the present invention may also be substantially free of any optional or a selected essential ingredient or feature described herein, provided that the remaining formula still contains all of the required ingredients or features as described herein. In this context, the term "substantially free" means that the selected composition contains less than a functional amount of the optional ingredient, typically less than 0.1% by weight, and also including zero percent by weight, of such optional or selected essential ingredient.

## Emulsion Viscosity

The nutritional emulsions of the present invention are oil-in-water emulsions that increase in viscosity following consumption, due primarily to the induced viscosity fiber system described hereinafter. These emulsions have a drinkable viscosity prior to consumption, referred to herein as the packaged viscosity, and a higher viscosity following consumption, referred to herein as the induced viscosity.

The induced viscosity provided by these nutritional emulsions helps reduce gastric emptying and the subsequent blood glucose response. The induced viscosity also provides a sense of fullness and enhanced satiety. These induced viscosity nutritional emulsions are especially useful in diabetics and in people interested in maintaining or losing weight.

As induced viscosity beverages, the nutritional emulsions of the present invention have a drinkable viscosity prior to consumption (i.e., packaged viscosity), which then increases in viscosity following consumption and upon entry into the stomach (i.e., induced viscosity). The viscosity increase arises primarily from the induced viscosity fiber system (described hereinafter) in the nutritional emulsion.

The nutritional emulsions of the present invention have a packaged viscosity of less than 300 cps, preferably from 40 to 250 cps, more preferably from 40 to 150 cps, including from 75 to 125 cps. In this context, and for purposes of defining the nutritional emulsions of the present invention, a packaged viscosity is measured after removing the emulsion from a sealed package such as from a retort processed or aseptically filled can, bottle, or other container. Viscosity measurements are taken using a Brookfield (model DVII+) viscometer with a 62 spindle at room temperature. The packaged viscosity is measured by operating the viscometer at a spindle speed that is the highest speed possible to obtain a reading that is on scale

The nutritional emulsions of the present invention are further defined by an induced viscosity of at least 300 cps, preferably at least 350 cps, including from 400 to 20,000 cps, and also including from about 800 to about 15,000 cps. For an emulsion comprising a polymer controlled induced viscosity fiber system, the induced viscosity is measured by adding 20 $\mu$L of bacterial alpha-amylase (Sigma) to 250 grams (g) of the emulsion, shearing the enzyme-treated emulsion for 30 minutes using a Glass-Col mixer, and then measuring viscosity using a Brookfield Viscometer (Model DV-II+) with a 62 spindle at room temperature. The induced viscosity is measured by operating the viscometer at a spindle speed that is the highest speed possible to obtain a reading that is on scale. This induced viscosity measurement is designed to assimilate the expected induced viscosity for the product following consumption and upon entry into the stomach.

## Low MSO Protein

The nutritional emulsions of the present invention comprise a protein source having 8% or less, on a molar basis, of the protein-bound methionine as protein-bound methionine sulfoxide, preferably from zero to 5%, including from 1 to 3%.

The nutritional emulsions are therefore preferably substantially free of any other protein source. In this context, the term "substantially free" means that the nutritional emulsions contains less than 0.5%, more preferably zero percent, by weight of such other protein source. Such other protein source is one having an MSO content greater than 8%.

The terms "MSO content", "methionine sulfoxide content" and "protein-bound methionine sulfoxide content" are used interchangeably herein to refer to the amount of protein-bound methionine sulfoxide, as a molar percentage of total protein-bound methionine, of a protein source. MSO content is determined by the analytical methods described herein.

It has been discovered that the induced viscosity nutritional emulsions of the present invention can be formulated with improved stability, and thus increased shelf life, provided that the protein source within the emulsion has a sufficiently low MSO content as defined herein. Improved stability is observed in the form of reduced creaming, reduced protein sedimentation, or both, as measured at 2 weeks, preferably at 6 weeks, more preferably at 6 months or longer, after initial formulation or packaging.

It has been discovered that, in preparing protein sources for use in nutritional products, varying amounts of the protein-bound methionine are oxidized to form protein-bound methionine sulfoxide moieties. It has been discovered that induced viscosity nutritional emulsions are unstable when subsequently formulated with a protein source having an MSO content above 8%, but are stable when the protein source has an MSO content of 8% or less.

Any protein source suitable for use in a nutritional product is also suitable for use herein, provided that it is processed or otherwise selected to have an MSO content of 8% or less. Suitable proteins sources from which to select or process suitable proteins include cows milk (e.g., casein, whey), animal (e.g., meat, fish), cereal (e.g., rice, corn), vegetable (e.g., soy), or combinations thereof.

Non limiting examples of suitable milk protein sources include milk protein isolates, casein protein isolates, milk protein concentrate, whole cows milk, partially or completely defatted milk, and so forth. Although milk protein isolates are preferred, it is also understood that not all such isolates will have an MSO content of 8% or less, so not all such isolates are suitable for use herein. The same is true for other protein sources as well.

Described below are some commercial protein sources and a corresponding MSO content (measured) for each. Those having an MSO content of 8% or less are suitable for use herein.

**Commercial Protein Sources**

|  | Supplier | Protein (commodity No.) | MSO Content |
|---|---|---|---|
| 1 | I | CaCaseinate | <5% - 73% |
| 2 | J | CaCaseinate | <5% |
| 3 | C | Ca Caseinate (No. 1970) | <1% |
| 4 | C | Na Caseinate (No 1980) | <1% |
| 5 | D | Casein hydrolyzate (No.2216) | 5% |
| 6 | E | Whey protein conc. (No. 1932) | 8 - 12% |
| 7 | F | Whey protein conc. (No. 15979) | 7 - 15% |
| 8 | G | Whey protein conc. (R0053 WPC) | 24 - 55% |
| 9 | H | Whey protein conc. (R0069 WPC) | 4 - 5% |
| 10 | G | Whey protein conc. (No. 1735) | 11 - 65% |
| 11 | C | Whey protein conc. (No. 18001) | 5% |
| 12 | I | Soy protein hydrolyzate (No. 3021) | 15% |
| 13 | A | Milk protein isolate (No. 15821) | 8 - 33% |
| 14 | C | Milk protein isolate | 2 - 3% |
| 15 | B | Milk protein isolate | 2% |
| 16 | I | Whey protein hydrolyzate (IF-3002 WPH) | 11 - 98% |
| 17 | I | Why protein hyrolyzate (IF-3002 WPH) | <3 - 21% |
| 18 | C | 1342 | <5% |
| 19 | K | 1922 | <5% |
| 20 | L | Mineral acid casein (No. 1380) | 1.7 - 2.4% |
| 21 | C | Mineral acid casein (No. 1380) | <1% |

(continued)

| | Supplier | Protein (commodity No.) | MSO Content |
|---|---|---|---|
| 22 | M | Mineral acid casein (No. 1380) | <1% |

Described in the following table are commercial nutritional products, each of which contains a protein source with a specified MSO content (as measured from finished product). None of the listed products, however, are induced viscosity emulsions comprising an induced viscosity fiber system.

**Commercial Nutritional Products**

| Product | Protein | MSO content |
|---|---|---|
| Alimentum® Abbott Laboratories | Casein protein hydrolyzate | 4% |
| Similac® with Iron (powder) Abbott Laboratories | Nonfat milk Whey protein conc. | 9% |
| Isomil® Powder Abbott Laboratories | Soy protein isolate | 5% |
| Good Start® Powder Nestle | Whey protein hydrolyzate | 44% |
| NAN HA1 Powder Nestle | Whey protein hydrolyzate | 10 - 11% |
| Enlive® Liquid Abbott Laboratories | Whey protein isolate | 23 - 32% |
| Glucerna® Shakes Abbott Laboratories | Milk protein isolate | 10 - 40% |
| Jevity® Nutrition Abbott Laboratories | Caseinate | <1% |

Since MSO content typically increases during processing, the MSO content for purposes of defining the present invention is preferably measured from finished product, although it is understood that the present invention also includes those finished products wherein the protein source has an MSO content of not more than 8% prior to formulation and processing, unless otherwise specified.

## Induced Viscosity Fiber System

The nutritional emulsions of the present invention comprise an induced viscosity fiber system, which includes any system that increases the viscosity of the emulsion following consumption, wherein the packaged viscosity and the induced viscosity of the emulsions following consumption are within the ranges as defined herein.

Any induced viscosity fiber system that is known or otherwise suitable for safe and effective oral administration are suitable for use herein, some examples of which are described in U.S. Patent Applications 20020193344, 20030125301, and 20030013679 (Wolf et al.).

Also suitable for use herein are the glucomannan compositions described in U.S. Patent 6,733,769 (Ryan et al.).

The induced viscosity fiber system is a polymer controlled induced viscosity fiber system such as that described in U.S. Patent Application 20030013679. Such a system comprises a neutral soluble fiber and a partially hydrolyzed starch having a degree of polymerization (DP) of at least 10.

The term "neutral water soluble fiber" as used herein refers to those fibers that can be dissolved in water at room temperature and that carry no charge at a neutral pH.

The nutritional emulsions of the present invention include those embodiments in which the weight ratio of the neutral soluble fiber to the partially hydrolyzed starch in the polymer controlled induced viscosity fiber system ranges from 0.35:5.0 to 1:5.0, including from 0.7:5.0 to 1:5.0, and also including 1:5.0.

Within an emulsion containing the polymer controlled induced viscosity fiber system, the neutral soluble fiber is maintained in a dispersed, insoluble state by the presence of the partially hydrolyzed starch. When two or more polymers such as these are present in the same solution, the solubility of the less soluble polymer (i.e., neutral soluble fiber) decreases as the concentration of the more soluble polymer (i.e., partially hydrolyzed starch) increases. However, when the partially hydrolyzed starch is digested by alpha amylase in the stomach, its increasing absence within the stomach allows the neutral soluble fiber within the consumed composition to solubilize and thus form a gel and a higher viscosity composition within the stomach. The resulting viscous mass in the stomach delays gastric emptying and slows or delays glucose absorption

Non limiting examples of neutral soluble fibers for use in the polymer controlled induced viscosity fiber system herein include guar gum, pectin, locust bean gum, methylcellulose, $\beta$-glucans, glucomannan, konjac flour, and combinations thereof. Preferred are glucomannan fiber, guar gum, and combinations thereof. The concentration of these neutral soluble fibers are typically at least 0.4%, including from 0.55 to 3.0 %, and also including from 0.65 to 1.5%, by weight of the nutritional emulsion.

Suitable partially hydrolyzed starches for use in this particular induced viscosity fiber system includes those having a DP of at least 10, preferably at least 20, including from 40 to 250, including from 60 to 120, and which are suitable for use in an oral nutritional product. In this context, the degree of polymerization (DP) is the number of glucose or monosaccharide units joined in the molecule. The concentration of the partially hydrolyzed starch is typically at least 2%, including from 3 to 20%, and also including from 3.5 to 6%, by weight of the nutritional emulsion

Non limiting examples of some suitable partially hydrolyzed starches for use herein include those obtained by acid hydrolysis, enzyme hydrolysis, or both. Preferred are those having a DP of from 40 to 250, including DP 100 maltodextrin, and other suitable polysaccharides such as inulin, hydrolyzed guar gum, gum Arabic, and combinations thereof. A DP value is the degree of polymerization of the partially hydrolyzed starch, i.e., the number of monosaccharide units in the partially hydrolyzed starch.

The partially hydrolyzed starch may also be characterized in terms of dextrose equivalents (DE) rather than DP values, wherein the partially hydrolyzed starch has a DE of less than 10, including from 1 to 8. A dextrose equivalent (DE) is a conventional measurement representing the average reducing power of maltodextrin or other polysaccharide as compared to a dextrose standard. DE values are derived from the formula $[DE = 100 \div DP]$, where DP is the degree of polymerization of the maltodextrin or other material, i.e., the number of monosaccharide units in the polysaccharide. For reference, glucose (dextrose) has a DE of 100; starch has a DE of approximately zero.

The induced viscosity fiber system herein includes those embodiments in which the neutral soluble fiber is glucomannan or konjac flour, and the partially hydrolyzed starch is one having a molecular weight of from 1,000 to 50,000 Daltons.

## Macronutrients

The nutritional emulsions of the present invention comprise fat, protein, and carbohydrate. As described herein, the protein must be a low MSO protein source and the carbohydrate must include an induced viscosity fiber system: The fat component is described hereinafter.

Although concentrations or amounts of each macronutrient in the nutritional emulsion of the present invention can vary dramatically depending upon the nutritional needs of the intended user, such concentrations or amounts most typically fall within one of the following embodied ranges.

| Macronutrient | Embodiments | | |
|---|---|---|---|
| | A | B | C |
| Carbohydrate[1] - % total calories | 10-85 | 20-60 | 40-60 |
| Fat - % total calories | 10-85 | 10-50 | 15-35 |
| Low MSO Protein - % total calories | 5-80 | 10-30 | 15-25 |
| | | | |
| Carbohydrate[1] g/100ml | 1-40 | 4-30 | 10-20 |
| Fat g/100ml | 0.1-30 | 0.5-15 | 1-5 |
| Low MSO Protein g/100ml | 0.5-30 | 1-15 | 2-10 |
| 1. Includes induced viscosity fiber system | | | |

The nutritional emulsions of the present invention comprise fat. Suitable fats or sources thereof include any that are known for or otherwise safe for use in oral nutritional products, non limiting examples of which include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, and combinations thereof.

The nutritional emulsions may comprise, as part of the fat component, polyunsaturated fatty acids, including polyunsaturated fatty acid esters or other natural or synthetic source, including short chain (less than about 6 carbon atoms per chain), medium chain (from about 6 to 18 carbon atoms per chain) and long chain (having at least about 20 carbon atoms per chain) fatty acids having two or more carbon:carbon double bonds, including n-3 (omega-3) and n-6 (omega-6) polyunsaturated fatty acids.

Non limiting examples of polyunsaturated fatty acids suitable for use herein include alpha-linolenic acid (ALA, C18:3n-3), stearidonic acid (C18:4n-3), eicosapentaenoic acid (EPA, C20:5n-3), docosapentaenoic acid (C22:5n-3), docosahexaenoic acid (DHA, C22:6n-3), linoleic acid (C18:2n-6), gamma-linolenic acid (GLA, C18:3n-6), eicosadienoic acid (C20:2n-6), arachidonic acid (ARA, C20:4n-6), di-homo-gamma-linolenic acid (DGLA, C20:3n-6), and combinations thereof.

The nutritional emulsions of the present invention may further comprise a carbohydrate in addition to that provided by the induced viscosity fiber system described herein. Non limiting examples of such additional carbohydrates include additional hydrolyzed or modified starch or cornstarch, glucose polymers, corn syrup, corn syrup solids, rice-derived carbohydrate, glucose, fructose, lactose, high fructose corn syrup, indigestible oligosaccharides (e.g., fructooligosaccharides), honey, sugar alcohols (e.g., maltitol, erythritol, sorbitol), and combinations thereof.

The nutritional emulsions of the present invention may further comprise any other nutrient, excipient, or other additive, provided that such optional ingredients are safe for use in an oral nutritional and do not unduly affect product performance. Vitamins and minerals are preferred in this regard.

## MSO Method

The methionine sulfoxide (MSO) content of the protein source for use herein is determined in accordance with the following method. For proper protein selection, the potential protein source is subjected to enzymatic hydrolysis at 37°C for 24 hours. The resulting enzyme digest is then evaluated for MSO and methionine content by reversed phase HPLC. The percentage of MSO in the protein source is then determined as follows:

$$\text{Protein-bound MSO, as mole \% of total protein-bound methionine} = \frac{MSO \times 100}{(MSO + MET)}$$

MSO = methionine sulfoxide in molar units

MET = methionine in same molar units

As an example of the above method, a protein source (calcium caseinate and sodium caseinate) is evaluated as follows:

### A. Standard Preparation

a. Dissolve 100 mg of L-methionine and 50 mg of DL-methionine sulfoxide (Fluka 64430) in 1000 mL of water. This is the High standard solution.

b. Dilute 25.0 mL of high standard solution to 50 mL with water. This is the low standard solution.

### B. Sample Preparation

a. Dissolve 90-95 mg of caseinate ingredient in 25 mL of 0.05M PIPES, pH 7.5, containing 0.1 % sodium azide.

b. Pipet 3.00 mL of the caseinate solution into a 1-dram vial.

c. Add 150 $\mu$L of pronase (Sigma P-5147), 2 mg/mL in 0.05M PIPES, pH 7.5, containing 0.1% sodium azide.

d. Add 60 $\mu$L of leucine aminopeptidase (Sigma L-0632), 2 mg/mL in water.

e. Add 30 $\mu$L of prolidase (Sigma P-6675), 2 mg/mL in water.

f. Cap vial and gently mix. Dispense 1600 $\mu$L into each of two crimp-seal HPLC autosampler vials (VWR 66020-953), and seal the vials.

g. Incubate at 37°C for 24 hours.

h. Test for MSO and for MET by the HPLC system described below.

### C. HPLC System

Column: ODS-AQ, 4.6 x 250 mm, 5$\mu$m, 120A, Waters AQ12S052546WT

Mobile Phase A: Water

Mobile Phase B: 350 mL 0.02 $KH_2PO_4$, pH 2.9; 650 mL acetonitrile

Temperature: 40°C

Detection: UV at 221 nm, 214 nm

Injection: 1 $\mu$L

Elution Program: See table in Appendix A under "C. HPLC System"

**Protein Samples**

MSO content of various commercial protein sources is measured and compared. The different protein sources are then evaluated for physical stability in an induced viscosity nutritional emulsion.

Of the nine protein sources tested, those having an MSO content of not more than 8% showed good emulsion stability, while those with an MSO content of less than 8% showed only fair or poor stability. These results illustrate the inversely proportional relationship between MSO content and HPLC Peak Height and between MSO content and induced viscosity emulsion stability. The data from the tests are summarized in the following table.

|   | Milk Protein Isolate Source code | MSO Content | HPLC Peak Height As mAU/g at 214 nm | Induced viscosity emulsion stability 55°C at 2 weeks |
|---|---|---|---|---|
| 1 | C 09 | 2.8% | 2381 | Good |
| 2 | C 15 | 2.9% | 2488 | Good |
| 3 | C 16 | 2.8% | 2397 | Good |
| 4 | C 18 | 2.5% | 2390 | Good |
| 5 | C 08 | 1.8% | 2325 | ND |
| 6 | B 04 | 2.1% | ND | Good |
| 7 | A 59 | 8% | 2014 | Good |
| 8 | A 04 | 13% | 1822 | Fair |
| 9 | A 17 | 33% | 1344 | Poor |

**EXAMPLES**

The following examples illustrate specific embodiments of the nutritional emulsions of the present invention, including suitable techniques to prepare the emulsions. The examples are given solely for the purpose of illustration and are not to be construed as limitations of the present invention.

**Example 1**

The following is an induced viscosity nutritional emulsion of the present invention and a process for making it. This embodiment contains a polymer controlled induced viscosity fiber system in combination with a protein having an MSO content in finished product of from 1-3%.

**Ingredients - Example 1**

| Fat blend | | Carbohydrate-mineral slurry | |
|---|---|---|---|
| High oleic safflower oil | 14.5 kg | Water | 341 kg |

| | | | |
|---|---|---|---|
| Canola oil | 13.7 kg | Maltodextrin DP100 | 45.3 kg |
| Soy lecithin | 0.7 kg | Fructose | 28 kg |
| Vitamin DEK premix | 47.3 g | Micronized tricalcium phosphate | 0.9 kg |
| Beta carotene 30% | 6.5 g | Magnesium chloride | 0.4 kg |
| Vitamin A palmitate | 4.6 g | Sodium citrate | 2.4 kg |
| Lutein | 4.9 g | Fructooligosaccharides | 12.6 kg |
| Vitamin E | 71.9 g | K phosphate dibasic | 0.35 kg |
| Konjac powder | 5.57 kg | Maltodextrin DP 5 | 21.1 kg |
| Guar gum | 3.33 kg | Maltitol syrup (70% solids) | 34.3 kg |
| | | Potassium Chloride | 1.2 kg |
| | | Calcium carbonate | 0.8 kg |
| | | Gellan gum | 0.2 kg |
| **Protein-in-water slurry** | | Mg phosphate dibasic | 3.1 kg |
| Water | 398 kg | Potassium citrate | 550 g |
| Milk Protein Isolate | 37.2 kg | Ultra/trace mineral premix | 270 g |
| Sodium caseinate | 3.24 kg | Potassium iodide | 0.16 g |
| Low viscosity Sodium Caseinate | 5.7 kg | Chromium chloride | 1.6 g |
| **Vitamin solution** | | | |
| Water | 39 kg | Water sol. vitamin premix | 70.9 g |
| Ascorbic acid | 425 g | Sucralose | 300 g |
| Choline chloride | 343 g | Vanilla flavor | 1.5 kg |

In preparing this nutritional emulsion (~1000 kg), the fat blend is formed separately by combining the specified ingredients. The protein-in-water slurry is also prepared separately by combining the specified ingredients. The carbohydrate/mineral slurry is likewise formed as a separate mixture by combining the specified ingredients.

The carbohydrate/mineral slurry is then added to the protein-in-water slurry and the blend pH adjusted to 6.7-7.0. To the resulting blend is added the fat blend. The mixture thus formed is then processed at UHT temperatures (295°F for 5 seconds) and homogenized at 4000 psi. The ingredients for the vitamin solution are then combined and the pH adjusted to 6.5-7.5 using 45% KOH. The vitamin solution is then added to the homogenized blend at standardization. The final blend is then packaged and sealed in individual 8 oz. containers and subjected to retort.

The resulting product has a packaged viscosity of 120 cps and an induced viscosity of over 14,000 cps (upon treatment with alpha amylase). The product remains stable without significant creaming or protein sedimentation at 2 weeks, 6 months, and 12 months.

## Example 2

The following is an induced viscosity nutritional emulsion of the present invention and a process for making it. This embodiment contains a polymer controlled induced viscosity fiber system in combination with a protein having an MSO content in finished product of from 1-3%.

Ingredients - Example 2

| Fat blend | | Carbohydrate-mineral slurry | |
|---|---|---|---|
| Diacylglycerol oil | 17.7 kg | Water | 341 kg |
| High oleic safflower oil | 9.4 kg | Maltodextrin DP100 | 45.3 kg |
| Canola oil | 1.5 kg | Fructose | 28 kg |
| Soy lecithin | 0.6 kg | Micronized tricalcium phosphate | 0.9 kg |
| Vitamin DEK premix | 47.3 g | Magnesium Chloride | 0.4 kg |
| Vitamin A palmitate | 4.6 g | Sodium citrate | 2.4 kg |
| Vitamin E | 71.9 g | Fructooligosaccharides | 12.6 kg |
| Konjac powder | 5.57 kg | K phosphate dibasic | 0.35 kg |
| Guar gum | 3.33 kg | Maltodextrin DP 5 | 21.1 kg |
| | | Maltitol syrup (70 %) | 34.3 kg |
| | | Potassium Chloride | 1.2 kg |
| | | Calcium carbonate | 0.8 kg |
| | | Gellan gum | 0.2 kg |
| | | Microcrystalline cellulose | 0.6 kg |
| Protein-in-water slurry | | Mg phosphate dibasic | 3.1 kg |
| Water | 398 kg | Potassium citrate | 550 g |
| Milk protein isolate | 30.6 kg | Ultra/trace mineral premix | 270 g |
| Sodium caseinate | 10 kg | Potassium iodide | 0.16 g |
| Low viscosity sodium caseinate | 5.7 kg | Chromium chloride | 1.6 g |

| Vitamin solution | | | |
|---|---|---|---|
| Water | 39 kg | Water sol. vitamin premix | 70.9 g |
| Ascorbic acid | 425 g | Sucralose | 300 g |
| Choline chloride | 343 g | Vanilla flavor | 1.5 kg |

In preparing this nutritional emulsion (~1000 kg), the fat blend is formed separately by combining the specified ingredients. The protein-in-water slurry is also prepared separately by combining the specified ingredients. The carbohydrate/mineral slurry is likewise formed as a separate mixture by combining the specified ingredients.

The carbohydrate/mineral slurry is then added to the protein-in-water slurry and the blend pH adjusted to 6.7-7.0. To the resulting blend is added the fat blend. The mixture thus formed is then processed at UHT temperatures (295°F for 5 seconds) and homogenized at 4000 psi. The ingredients for the vitamin solution are then combined and the pH adjusted to 6.5-7.5 using 45% KOH. The vitamin solution is then added to the homogenized blend at standardization. The final blend is then packaged and sealed in individual 8 oz. containers and subjected to retort.

The resulting product has a packaged viscosity of 120 cps and an induced viscosity of over 14,000 cps (upon treatment with alpha amylase). The product remains stable without significant creaming or protein sedimentation at 2 weeks, 6 months, and 12 months.

## Example 3

The following is an induced viscosity nutritional emulsion of the present invention and a process for making it. This embodiment contains a polymer controlled induced viscosity fiber system in combination with a protein having an MSO content in finished product of from 1-3%.

**Ingredients - Example 3**

| Fat blend | | Carbohydrate-mineral Slurry | |
|---|---|---|---|
| Diacylglycerol oil | 8.8 kg | Water | 341 kg |
| High oleic safflower oil | 12.9 kg | Maltodextrin DP100 | 45.3 kg |
| Canola oil | 5.5 kg | Fructose | 28 kg |
| Soy lecithin | 2.2 kg | Micronized tricalcium phosphate | 0.9 kg |
| Vitamin DEK premix | 47.3 g | Magnesium chloride | 0.4 kg |
| Vitamin A palmitate | 4.6 g | Sodium citrate | 2.4 kg |
| Vitamin E | 71.9 g | Fructooligosaccharides | 12.6 kg |
| Konjac powder | 5.57 kg | K phosphate dibasic | 0.35 kg |
| Guar gum | 3.33 kg | Maltodextrin DP 5 | 21.1 kg |

| | | | |
|---|---|---|---|
| | | Maltitol syrup (70% solids) | 34.3 kg |
| | | Potassium chloride | 1.2 kg |
| | | Calcium carbonate | 0.8 kg |
| | | Gellan gum | 0.2 kg |
| Protein-in-water slurry | | Mg phosphate dibasic | 3.1 kg |
| Water | 398 kg | Potassium citrate | 550 g |
| Whey protein | 8.1 kg | Ultra/trace mineral premix | 270 g |
| Sodium caseinate | 4.4 kg | Potassium iodide | 0.16 g |
| Calcium caseinate | 21.7 kg | | |
| Low viscosity sodium caseinate | 11 kg | Chromium chloride | 1.6 g |
| Vitamin solution | | | |
| Water | 39 kg | Water sol. vitamin premix | 70.9 g |
| Ascorbic acid | 425 g | Sucralose | 300 g |
| Choline chloride | 343 g | Vanilla flavor | 1.5 kg |

In preparing this nutritional emulsion (~1000 kg), the fat blend is formed separately by combining the specified ingredients. The protein-in-water slurry is also prepared separately by combining the specified ingredients. The carbohydrate/mineral slurry is likewise formed as a separate mixture by combining the specified ingredients.

The carbohydrate/mineral slurry is then added to the protein-in-water slurry and the blend pH adjusted to 6.7-7.0. To the resulting blend is added the fat blend. The mixture thus formed is then processed at UHT temperatures (295°F for 5 seconds) and homogenized at 4000 psi. The ingredients for the vitamin solution are then combined and the pH adjusted to 6.5-7.5 using 45% KOH. The vitamin solution is then added to the homogenized blend at standardization. The final blend is then packaged and sealed in individual 8 oz. containers and subjected to retort.

The resulting product has a packaged viscosity of 120 cps and an induced viscosity of over 14,000 cps (upon treatment with alpha amylase). The product remains stable without significant creaming or protein sedimentation at 2 weeks, 6 months, and 12 months.

Claims

1. A polymer-controlled induced viscosity nutritional emulsion comprising

(A) protein having a protein-bound methionine sulfoxide content that is 8% or less of the total protein-bound methionine, on a molar basis,
(B) fat, and
(C) a polymer-controlled induced viscosity fiber system that provides the emulsion with a packaged viscosity of less than 300 cps and an induced viscosity following consumption of at least 300 cps,

wherein the induced viscosity nutritional emulsion is an oil-in-water emulsion.

2. The nutritional emulsion of claim 1 wherein the emulsion comprises, as a percentage of total calories, from 10 to 85% carbohydrate, from 10 to 85% fat, and from 5 to 40% protein.

**3.** The nutritional emulsion of claim 2 wherein the protein-bound methionine sulfoxide content is from 1 to 5% of the total protein-bound methionine, on a molar basis.

**4.** The nutritional emulsion of claim 1 wherein the induced viscosity fiber system comprises, by weight of the emulsion, at least 0.4% of a neutral soluble fiber and at least 2% of a partially hydrolyzed starch having a degree of polymerization of at least 10.

**5.** The nutritional emulsion of claim 4 wherein the emulsion comprises from 0.55 to 3.0% by weight of the neutral soluble fiber and from 2 to 6% by weight of the partially hydrolyzed starch.

**6.** The nutritional emulsion of claim 5 wherein the neutral soluble fiber is selected from the group consisting of guar gum, high-methoxy pectin, locust bean gum, methylcellulose, beta-glucans, glucomannan, konjac flour, and combinations thereof.

**7.** The nutritional emulsion of claim 6 wherein the partially hydrolyzed starch has a degree of polymerization of from 40 to 250.

**8.** The nutritional emulsion of claim 1 wherein the emulsion has a packaged viscosity of from 40 to 250 cps and an induced viscosity of from 400 to 20,000 cps.

**Patentansprüche**

**1.** Eine Nahrungsemulsion mit polymergesteuerter induzierter Viskosität, die Folgendes umfasst:

(A) Protein mit einem Gehalt an proteingebundenem Methionin, der 8% oder weniger des gesamten proteingebundenen Methionins, auf molarer Basis, beträgt,
(B) Fett, und
(C) ein Fasersystem mit polymergesteuerter induzierter Viskosität, das der Emulsion eine verpackte Viskosität von weniger als 300 cps und eine induzierte Viskosität nach dem Verbrauch von mindestens 300 cps verleiht,

worin die Nahrungsemulsion mit induzierter Viskosität eine Öl-in-Wasser-Emulsion ist.

**2.** Die Nahrungsemulsion gemäß Anspruch 1, worin die Emulsion, als Prozentsatz der Gesamtkalorien, von 10 bis 85% Kohlenhydrat, von 10 bis 85% Fett und von 5 bis 40% Protein umfasst.

**3.** Die Nahrungsemulsion gemäß Anspruch 2, worin der Gehalt an proteingebundenem Methioninsulfoxid von 1 bis 5% des gesamten proteingebundenen Methionins auf molarer Basis beträgt.

**4.** Die Nahrungsemulsion gemäß Anspruch 1, worin das Fasersystem mit induzierter Viskosität nach Gewicht der Emulsion mindestens 0,4% einer neutral löslichen Faser und mindestens 2% einer partiell hydrolysierten Stärke mit einem Polymerisationsgrad von mindestens 10 umfasst.

**5.** Die Nahrungsemulsion gemäß Anspruch 4, worin die Emulsion von 0,55 bis 3,0 Gewichtsprozent der neutral löslichen Faser und von 2 bis 6 Gewichtsprozent der partiell hydrolysierten Stärke umfasst.

**6.** Die Nahrungsemulsion gemäß Anspruch 5, worin die neutral lösliche Faser gewählt ist aus der Gruppe bestehend aus Guargummi, Pektin mit hohem Methoxygehalt, Johannisbrotgummi, Methylcellulose, Beta-Glucanen, Glucomannan, Konjakmehl und Kombinationen davon.

**7.** Die Nahrungsemulsion gemäß Anspruch 6, worin die partiell hydrolysierte Stärke einen Polymerisationsgrad von 40 bis 250 hat.

**8.** Die Nahrungsemulsion gemäß Anspruch 1, worin die Emulsion eine verpackte Viskosität von 40 bis 250 cps und eine induzierte Viskosität von 400 bis 20.000 cps hat.

**Revendications**

1. Émulsion alimentaire à viscosité induite régulée par polymère comprenant

   (A) une protéine ayant une teneur en sulfoxide de méthionine lié à la protéine qui est inférieure ou égale à 8 % de la méthionine liée à la protéine totale, sur une base molaire,
   (B) de la graisse, et
   (C) un système de fibres à viscosité induite régulée par polymère qui confère à l'émulsion une viscosité de fabrication inférieure à 300 cps et une viscosité induite après consommation d'au moins 300 cps,

   dans laquelle l'émulsion alimentaire à viscosité induite est une émulsion d'huile dans l'eau.

2. Émulsion alimentaire selon la revendication 1 dans laquelle l'émulsion comprend, en pourcentage des calories totales, de 10 à 85 % de glucide, de 10 à 85 % de graisse, et de 5 à 40 % de protéine.

3. Émulsion alimentaire selon la revendication 2 dans laquelle la teneur en sulfoxyde de méthionine lié à la protéine est de 1 à 5 % de la méthionine liée à la protéine totale, sur une base molaire.

4. Émulsion alimentaire selon la revendication 1 dans laquelle le système de fibres à viscosité induite comprend, en poids de l'émulsion, au moins 0,4 % d'une fibre soluble neutre et au moins 2 % d'un amidon partiellement hydrolysé ayant un degré de polymérisation d'au moins 10.

5. Émulsion alimentaire selon la revendication 4 dans laquelle l'émulsion comprend de 0,55 à 3,0 % en poids de la fibre soluble neutre et de 2 à 6 % en poids de l'amidon partiellement hydrolysé.

6. Émulsion alimentaire selon la revendication 5 dans laquelle la fibre soluble neutre est choisie dans le groupe constitué de la gomme de guar, de la pectine à forte teneur en méthoxy, de la gomme de caroube, de la méthylcellulose, des bâta-glucanes, du glucomannane, de la farine de konjac et de leurs combinaisons.

7. Émulsion alimentaire selon la revendication 6 dans laquelle l'amidon partiellement hydrolysé a un degré de polymérisation de 40 à 250.

8. Émulsion alimentaire selon la revendication 1 dans laquelle l'émulsion a une viscosité de fabrication de 40 à 250 cps et une viscosité induite de 400 à 20 000 cps.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020193344 A, Wolf
- US 20030013679 A, Wolf
- US 20030125301 A
- US 6733769 B, Ryan